Europäisches Patentamt

European Patent Office

Office européen des brevets

(19)

(11) Publication number : **0 469 810 A1**

(12) # EUROPEAN PATENT APPLICATION

(21) Application number : **91306899.5**

(22) Date of filing : **29.07.91**

(51) Int. Cl.⁵ : **C07K 13/00,** C07K 3/20, C12N 15/52, C12N 15/29

(30) Priority : **30.07.90 US 559939**

(43) Date of publication of application :
**05.02.92 Bulletin 92/06**

(84) Designated Contracting States :
**DE FR GB IT**

(71) Applicant : **IOWA STATE UNIVERSITY RESEARCH FOUNDATION, INC.**
**214 O & L**
**Ames, Iowa 50011-3020 (US)**

(72) Inventor : **Nikolau, Basil J.**
**3425 Oakland Ames**
**Iowa 50010 (US)**
Inventor : **Wurtele, Eve Syrkin**
**3425 Oakland Ames**
**Iowa 50010 (US)**

(74) Representative : **Bassett, Richard Simon et al**
**ERIC POTTER & CLARKSON St. Mary's Court**
**St. Mary's Gate**
**Nottingham NG1 1LE (GB)**

(54) **Plant acetyl-coa carboxylase polypeptide and gene.**

(57)   A purified, biotin-containing polypeptide with a molecular weight of about 50 kilodaltons is disclosed herein. The polypeptide is a subunit of plant acetyl-CoA carboxylase, which catalyzes the carboxylation of plant acetyl-CoA to form plant malonyl-CoA. Modified polypeptides are also disclosed herein as well as methods for obtaining the polypeptides. Also disclosed herein are DNA sequences coding for the polypeptides and methods of obtaining a preferred cDNA sequence by screening a cDNA expression library with antibodies to the 50kD polypeptide.

EP 0 469 810 A1

FIELD OF THE INVENTION

This invention relates to plant acetyl-CoA carboxylase. In particular, it relates to a purified polypeptide subunit of the enzyme and to DNA coding for the polypeptide. The DNA may be used in methods and vectors for transforming plant cells and plants for the purpose of studying and eventually modifying fatty acid synthesis in plants.

REFERENCES

Several publications are referenced herein by Arabic numerals within parenthesis. Full citations for these references may be found at the end of the specification immediately preceding the claims. The disclosures of these publications are hereby incorporated herein by reference in their entirety, unless otherwise noted.

BACKGROUND OF THE INVENTION

Acetyl-CoA carboxylase is a biotin-containing enzyme that catalyzes the ATP-dependent carboxylation of acetyl-CoA to form malonyl-CoA. This enzyme has been extensively investigated from a wide diversity of organisms, since it catalyzes the initial reaction of fatty acid biosynthesis. This reaction is commonly believed to be the rate-limiting step in this biosynthetic pathway. Fatty acids are important constituents of all cells, being the building blocks from which various lipids are assembled that are utilized for cellular membranes. Furthermore, fatty acids produced in seeds of plants are assembled into triglycerides that are the source of vegetable oils that are commercially used as a source of dietary fat and for industrial purposes. These oils include soy oil, canola oil, sunflower oil, and corn oil. Having the gene for acetyl-CoA carboxylase would provide the opportunity to alter fatty acid synthesis in plants.

In contrast to the large volume of work on this enzyme from non-plant species, our understanding of plant acetyl-CoA carboxylase has been relatively meager and confused. It is believed that the naturally occurring enzyme has a molecular weight of approximately 500kD. However, it has been difficult to study and characterize this enzyme because it has been virtually impossible to purify the enzyme in its complete and active form. Such efforts have almost invariably resulted in it becoming denatured or degraded into various subunits and fragments. The molecular weights of these degradation products have shown a tremendous variability.

For example, Hellyer, et al., Biochemical Society Transactions, 14:565-568 (1986), incorporated herein by reference, reports that efforts to isolate and characterize the subunit structure of plant acetyl-CoA carboxylase have not yielded uniform results. Table 1 of the paper discloses polypeptide molecular weights ranging from 21kD to 240kD. These include a subunit from maize leaf at 60-61kD, one from avocado at 57kD, another from avocado at 47kD, and subunits from wheat germ ranging from 21.5-135kD.

At least one of these polypeptides has been purified. Nikolau, et al., Arch. Biochem. and Biophy., 228:86-96 (1984), incorporated herein by reference, discloses the purification of the enzyme from maize leaf homogenates by precipitation with polyethylene glycol and ammonium sulfate followed by gel filtration. The authors estimated the molecular weight of the native enzyme to be about 500kD. Electrophoresis in SDS-PAGE revealed a single subunit with a molecular weight of 60-61kD.

Nevertheless, a consensus appears to have emerged that the natural enzyme has subunits with a molecular weight of approximately 220kD. For example, Slabas, et al., Plant Science, 39:177-182 (1985), incorporated herein by reference, reports the purification of a 220kD polypeptide subunit of the enzyme from rape seed. Egin-Buhler, et al., Eur. J. Biochem., 133:335-339 (1983), incorporated herein by reference, reports the purification of a 220kD polypeptide subunit of parsley acetyl-CoA carboxylase. A recent review of the literature, Harwood, Ann. Rev. Plant. Physiol. Plant Mol. Bio., 39:101-138 (1988), incorporated herein by reference, reviews the scientific efforts to purify plant acetyl-CoA carboxylase on pages 101-105. It notes the work mentioned above of Nikolau, et al. with maize. However, it focuses on the work of others who found polypeptides in the range of 210-240kD, and it concludes on page 105 that "it seems probable that plant acetyl-CoA carboxylases are all high molecular-mass multi-functional proteins."

Part of the confused state of the understanding of plant acetyl-CoA carboxylase may be due to the fact that the metabolic function of this enzyme is not limited to fatty acid biosynthesis in plants. Malonyl-CoA, a product of the acetyl-CoA carboxylase reaction, is an intermediate in the biosynthesis of at least seven different classes of phytochemicals. The diversity in the metabolic function of acetyl-CoA carboxylase in plants has led to the suggestion that these organisms contain more than one form of this enzyme.

Consistent with this hypothesis, plants were found to contain multiple biotin-containing polypeptides, even though acetyl-CoA carboxylase was the only known biotin-containing enzyme in these organisms. This work was reported in Nikolau, et al., Plant Physiol., 75:895-901 (1984), incorporated herein by reference. The paper discloses the use of Western blotting of proteins from plant leaves fractionated by SDS-PAGE electrophoresis, using $^{125}$I-streptavidin. Four biotinylated polypeptides were found, having molecular weights of 62kD, 51kD, 34kD, and 32kD.

The authors noted that the 62kD polypeptide had been identified as the biotin-containing subunit of acetyl-CoA carboxylase in maize, and they speculated that the other polypeptides might be subunits of isozymes of the enzyme.

Subsequently, Wurtele, et al., Arch. Biochem. and Biophys., 278:179-186 (1990), incorporated herein by reference, reported the visualization of six biotin-containing polypeptides in proteins extracted from carrot somatic embryos through Western analysis with $^{125}$I-streptavidin. The polypeptides had relative molecular masses of 210kD, 140kD, 73kD, 50kD, 39kD, and 34kD. The authors noted that the 210kD molecular weight polypeptide compared favorably to acetyl-CoA carboxylases with biotinylated subunits of approximately this molecular weight that had been purified from plants, yeasts, and animals. They also noted that the 73kD polypeptide compared favorably with another form of the enzyme purified from corn leaves with the reported biotinylated subunit having a molecular weight of approximately 62kD. The authors explained the multiplicity of the biotin-containing polypeptides by their discovery of three other biotin-containing proteins in the extracts besides acetyl-CoA carboxylase. These enzymes were pyruvate carboxylase, propionyl-CoA carboxylase, and methylcrotonyl-CoA carboxylase, which are known in other organisms but had not been reported previously in plants.

Perhaps one of the major reasons for the confusion in the scientific literature has been the difficulty of purifying the natural enzyme. Numerous unsuccessful attempts have been made to purify the complete enzyme in active form so that it could be carefully studied and characterized. Even in the very few cases where it has been purified in its complete and active form, the amount has been exceedingly small. It was not sufficient for the production of antibodies. Such antibodies would provide the basis for obtaining the gene.

Investigators generally have had to settle with the purification and study of subunits. One serious drawback of this approach is always the uncertainty and possibility that what is believed to be a subunit of the larger protein may, in fact, only be a degradation product.

It is well known that avidin and streptavidin bind to biotin. This fact has been used in techniques to purify biotin-containing molecules. For example, Rylatt, et al., Arch. Biochem. and Biophys., 183:113-122 (1977), incorporated herein by reference, discloses the isolation of biotin-containing tryptic peptides of pyruvate carboxylase using a column containing avidin bound to a solid substrate. The problem with this approach is that avidin and streptavidin work too well in the sense that they bind so tightly to the biotin-containing molecule that it is extremely difficult to remove the molecule. Removal requires extremely harsh conditions, which results in the denaturating and occasional degradation of the protein.

Avidin and streptavidin occur naturally in a tetrameric form. Several years ago it was recognized that the use of only one of the subunits of avidin, i.e., the use of avidin in its monomeric form, would result in biotin-containing polypeptides being more loosely bound in affinity columns. For example, Gravel, et al. Arch. Biochem. and Biophy., 201:669-673 (1980), incorporated herein by reference, reports that the purification of biotin-containing carboxylases by avidin has been difficult to achieve because avidin binds biotin so tightly that it has not been possible to elute the active enzyme. The article further stated that this difficulty had been partially overcome by using monomeric avidin, since it has a reduced affinity for biotin. The authors reported the favorable use of this approach to purify a different biotin-containing enzyme, propionyl-CoA carboxylase.

Even though the use of monomeric avidin still did not permit the purification of a complete and active form of plant acetyl-CoA carboxylase, it became the method of choice for studying this enzyme through the isolation or purification of its subunits. For example, Egin-Buhler, et al., op. cit., and Slabas, et al., op. cit., both reported the purification of a 220kD polypeptide subunit of acetyl-CoA carboxylase through the use of monomeric avidin affinity chromatography.

Unfortunately, this currently preferred purification technique and others still have one major drawback. It has not been possible to obtain a sufficient amount of the enzyme or a subunit to fully characterize it and, perhaps more importantly, to obtain the gene coding for it.

The inventors realized that it was not necessary to obtain active enzyme in order to obtain the gene and thereby characterize the protein by characterizing the gene. Accordingly, they used tetrameric rather than monomeric avidin to obtain a sufficient amount of a 50kD polypeptide subunit of the enzyme to raise antibodies in rabbits, even though it was in inactive form. The antibodies were then used to screen a cDNA expression library for the purpose of isolating the cDNA that codes for the polypeptide. Having this DNA will offer the opportunity of altering oil seed quality and quantity in oil seed crops, enhancing herbicide resistance in certain types of plants, and improving transformation technology for certain plant cells. It also offers the opportunity to learn more about important biochemical pathways in plants.

SUMMARY OF THE INVENTION

It is an object of the present invention to provide a purified polypeptide that is a subunit of plant acetyl-CoA carboxylase.

It is a further object of the invention to provide other polypeptides that catalyze the carboxylation of acetyl-CoA to form malonyl-CoA.

A further object of the invention is to provide a method of obtaining the polypeptides of the invention.

Another object of the invention is to provide a method of obtaining biotin-containing polypeptides in isolation from non-biotin-containing polypeptides from plants.

Yet another object of the invention is to provide an isolated or substantially purified DNA sequence that codes for the polypeptides of the invention and methods of obtaining such sequences.

A still further object of the invention is to provide for related cDNA sequences, recombinant DNA sequences, expression vectors, host cells transformed by the DNA of the invention, and the recombinant polypeptide produced by the transformed cells.

Additional objects and advantages of the invention will be set forth in part in the description which follows, and in part will be obvious from the description, or may be learned by the practice of the invention. The objects and advantages of the invention will be attained by means of the instrumentalities and combinations particularly pointed out in the appended claims.

To achieve the objects and in accordance with the purpose of the invention, as embodied and broadly described herein, the present invention provides a purified biotin-containing polypeptide with a molecular weight of about 50 kilodaltons. The polypeptide is a subunit of plant acetyl-CoA carboxylase. The invention further provides polypeptides derived from this polypeptide which catalyze the carboxylation of acetyl-CoA to form malonyl-CoA.

In another embodiment, the invention comprises a method for obtaining biotin-containing polypeptides in isolation from non-biotin-containing polypeptides from plants. The proteins in the plant tissue containing these polypeptides are extracted with a protein-extracting solution. The separated extract is then contacted with tetrameric avidin or tetrameric streptavidin bound to a solid support for a sufficient time and under appropriate conditions to bind the biotin-containing polypeptides to the avidin or streptavidin. The polypeptides are then removed and recovered in isolated form. Preferably, the polypeptides are purified by separating them by their molecular weight and recovering them in purified form. Most preferably, the method is applied to plant tissue containing acetyl-CoA carboxylase, and the 50kD purified polypeptide is recovered.

In an alternative preferred embodiment, the invention comprises a method of obtaining plant acetyl-CoA carboxylase in purified form. The extract is contacted with antibodies to the polypeptide for a sufficient time and under appropriate conditions to bind the acetyl-CoA carboxylase to the antibodies. The bound complexes are then separated from the extract. The acetyl-CoA carboxylase is removed from the antibodies and recovered in purified form. Preferably, the antibodies are bound to a solid support.

The polypeptide is used to obtain the DNA of the invention. Preferably, this DNA is complementary DNA (cDNA). Antibodies to the polypeptide are used to screen a cDNA expression library. The library comprises clones of vectors into which different sequences of plant cDNA have been operably and recoverably inserted. Each of the vectors contains only one sequence of the cDNA. Contacting the antibodies with the clones of the library results in the binding of the antibodies to those clones expressing the polypeptide. The clones are isolated, and the cDNA is thereby obtained. The cDNA sequence can then be recovered from the clone.

Having the cDNA sequence permits the construction of a probe that can be used to screen a genomic library. This will permit the isolation of the gene coding for the polypeptide. Accordingly, another embodiment of the invention comprises DNA coding for the 50kD polypeptide. The DNA of the invention further includes derived DNA obtained by the application of standard techniques.

In yet another embodiment of the invention, the DNA is operably linked to appropriate regulatory control nucleic acid sequences that are capable of effecting expression of the DNA sequence in transformed host cells. Preferably, such sequences comprise an expression vector. This DNA is inserted into procaryotic or eucaryotic cells, preferably plant cells, in order to transform the cells. The cells then produce a recombinant biotin-containing polypeptide with a molecular weight of about 50kD, which preferably catalyzes the carboxylation of acetyl-CoA to form malonyl-CoA.

This ability to genetically engineer plant cells and plants will permit a greater understanding of fatty acid synthesis in plants and the eventual ability to modify such synthesis.

The accompanying drawings, which are incorporated in and constitute a part of this specification, illustrate one embodiment of the invention and, together with the description, serve to explain the principles of the invention.

## BRIEF DESCRIPTION OF THE DRAWINGS

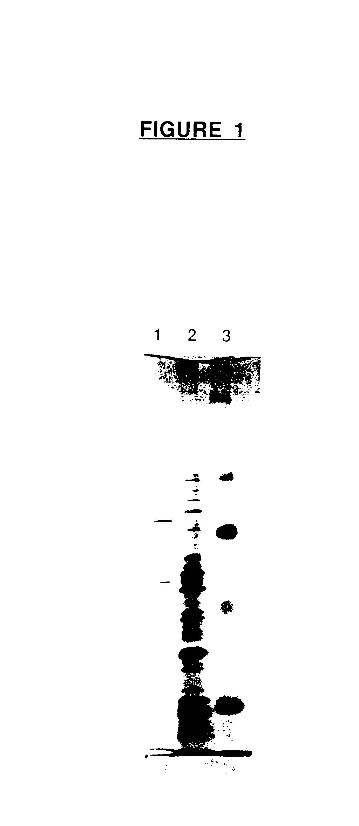

Figure 1 shows SDS-PAGE analysis of purified biotin-containing polypeptides from carrot somatic embryos. A protein extract prepared with boiling 2% SDS from 500g of carrot somatic embryos (lane 2) was applied to a column of Sepharose-tetrameric avidin. Following extensive washing to eliminate unbound proteins, the biotin-containing polypeptides were eluted with boiling 2% SDS (lane 1). Lane 1: 20µg of purified biotin-containing polypeptides. Lane 2: 30µg of protein from the embryo extract. Lane 3: Molecular weight standards in descending order are myosin (200Kd), phosphorylase b (97.4Kd), bovine serum albumin (68Kd), ovalbumin (43Kd), and carbonic anhydrase (29Kd).

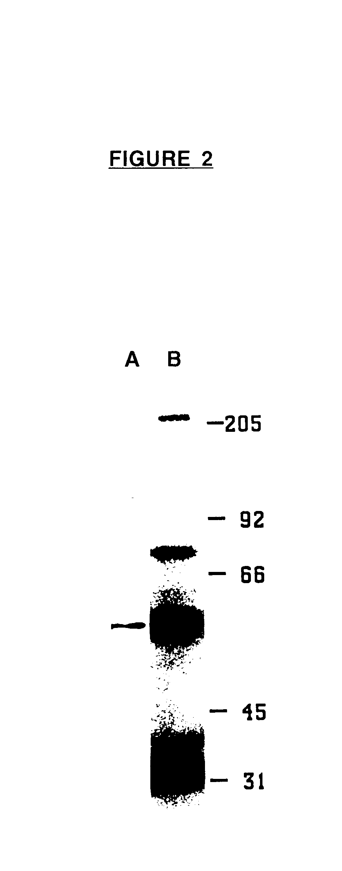

Figure 2 shows Western analysis of the 50kD biotin-containing polypeptide. Two identical aliquots of protein extracts (30μg each) from somatic embryos of carrot were subjected to SDS-PAGE and transferred to nitrocellulose. Filter A was sequentially incubated with antiserum to the 50Kd biotin-containing polypeptide and $^{125}$I-protein A. Filter B was incubated with $^{125}$I-streptavidin to show all the biotin-containing polypeptides in the extract.

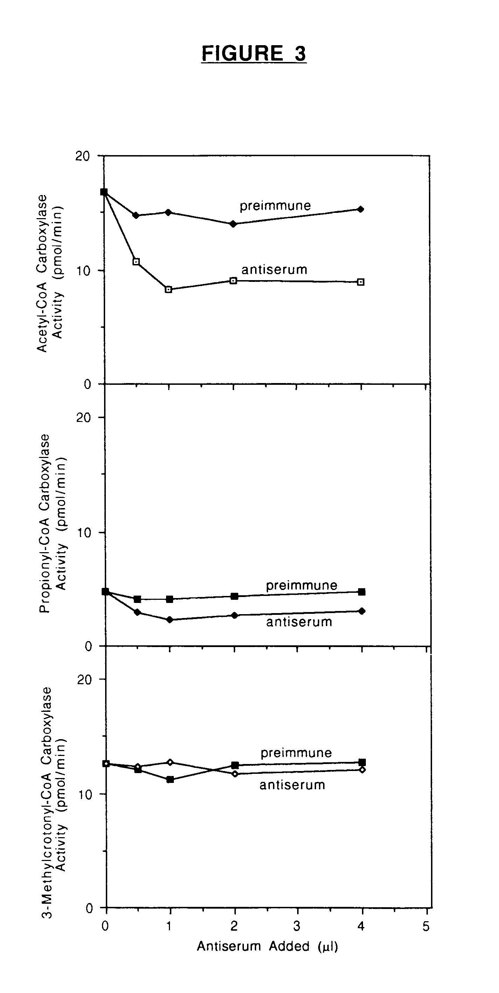

Figure 3 shows the effect of pre-immune serum and antiserum on the activity of three biotin-containing plant enzymes. Increasing amounts of pre-immune serum or antiserum to the 50kD biotin-containing polypeptide were added to aliquots of an extract from developing somatic embryos of carrot. Following an incubation for 1 hour on ice, a suspension of Agarose-Protein A was added, and the mixture was incubated for an additional hour on ice. The mixture was then centrifuged at 12,000g for 2 minutes to remove the antigen-antibody-Protein A complexes. The supernatant was retained and assayed for residual acetyl-CoA carboxylase, propionyl-CoA carboxylase, and 3-methylcrotonyl-CoA carboxylase , activities.

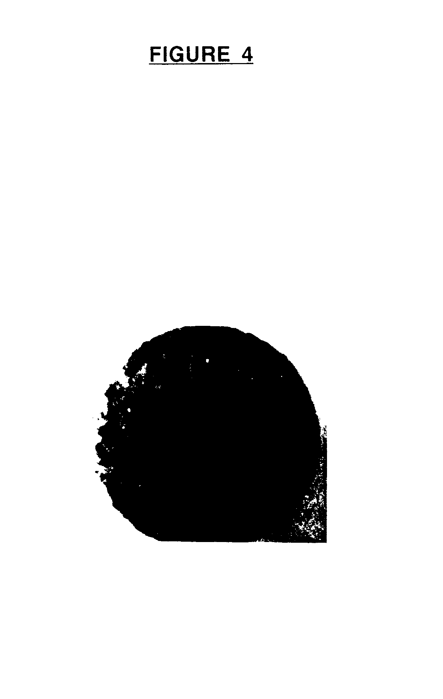

Figure 4 relates to the immunological screening of an expression cDNA library with antiserum to the 50Kd biotin-containing polypeptide. A replica of lambda plaques on a nitrocellulose filter was sequentially incubated with antiserum to the 50Kd biotin-containing polypeptide and $^{125}$I-Protein A. Putative recombinant phage representing the cDNA clone CC6 show a positive signal.

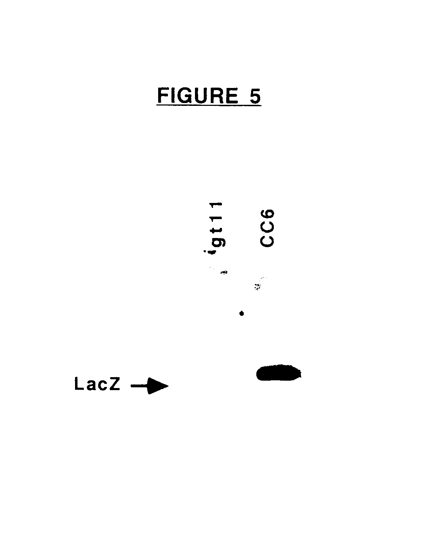

Figure 5 shows Western confirmation of the identity of the clone CC6. Recombinant lambda from a plaque putatively identified as coding for the 50Kd biotin-containing polypeptide (CC6) and wild-type lambda gt11 phage were utilized to prepare lysogens in E. coli. Y1089 cells, lysates from IPTG induced cultures of such lysogens, were analyzed by SDS-PAGE and Western analysis. The blot was sequentially incubated with antiserum to the 50kD biotin-containing polypeptide and $^{125}$I-Protein A. The position of the authentic LacZ protein is indicated.

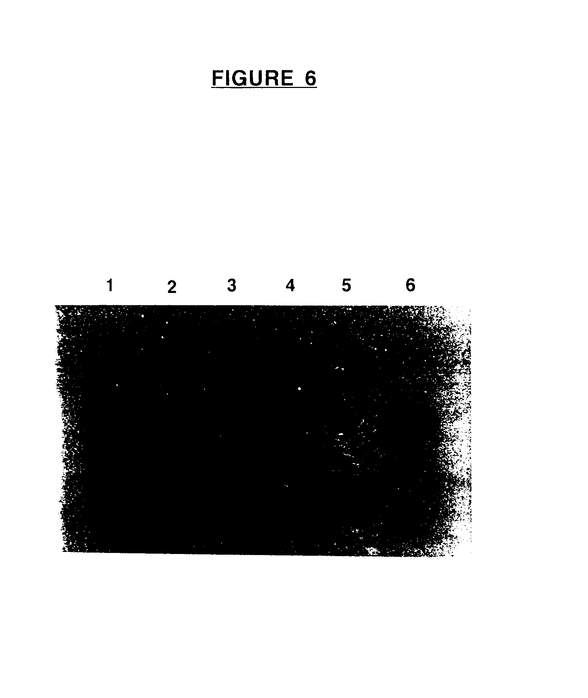

Figure 6 shows Northern analysis of the RNA coding for the 50kD biotin-containing polypeptide. RNA was isolated from embryos at the globular (lane 1), heart (lane 2), torpedo (lane 3), and germinating stages (lane 4) of development and from leaves (lane 5) and roots (lane 6). Equal amounts (10μg) of these RNA preparations were subjected to electrophoresis in a formaldehyde-containing agarose gel, transferred to a nitrocellulose filter and hybridized with $^{32}$P-labelled pCC6. A single RNA band was detected that is 2000 bases in length.

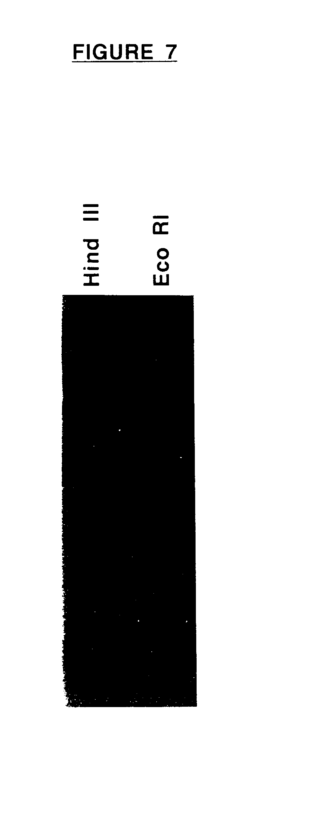

Figure 7 shows Southern analysis of carrot genomic DNA. Carrot genomic DNA was digested with the enzymes HindIII and EcoRI and subjected to electrophoresis. Following transfer to a nitrocellulose filter the DNA was hybridized with $^{32}$P-labelled pCC6. Six DNA restriction fragments hybridize to this clone, indicating that the genes for the 50kD biotin-containing polypeptide are present as a small gene family in the carrot genome.

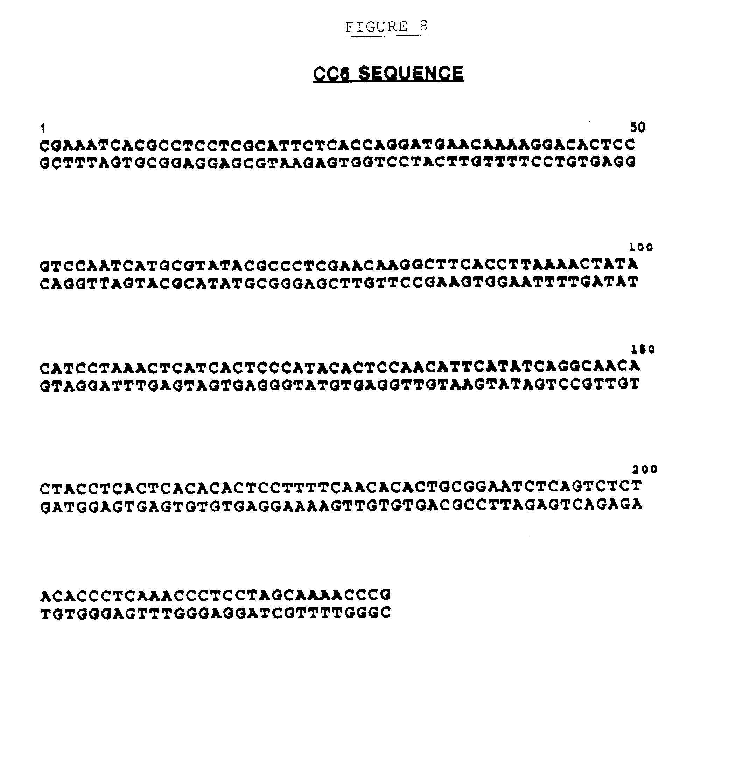

Figure 8 shows the DNA sequence of CC6. The cDNA insert was subcloned into the Eco R1 site of the plasmid pUC19 and sequenced by the procedure of Sanger, et al. The sequence of both strands is shown with the numerical order of the bases shown above the sequence.

DETAILED DESCRIPTION OF THE INVENTION

Reference will now be made in detail to the presently preferred embodiments of the invention, which, together with the following examples, serve to explain the principles of the invention.

The invention relates to a polypeptide which is a subunit of plant acetyl-CoA carboxylase and to DNA coding for the polypeptide. The polypeptide is a purified, biotin-containing polypeptide with a molecular weight of about 50 kilodaltons. As used herein, the term "purified" and variants thereof refers to polypeptides that have been isolated from other polypeptides naturally associated with the polypeptide of the invention. The purified polypeptide is at least 97% pure by weight and preferably at least 99% pure by weight. That is, a composition comprising the purified polypeptide contains no more than 3% and preferably no more than 1% by weight of proteins or polypeptides that are not the polypeptide of the invention.

The polypeptide of the invention has activity similar to that of plant acetyl-CoA carboxylase. That is, it catalyzes the carboxylation of plant acetyl-CoA to form plant malonyl-CoA, and, in fact, should catalyze the carboxylation of acetyl-CoA from any organism to malonyl-CoA.

In a preferred embodiment, the polypeptide is isolated from plant tissue that contains acetyl-CoA carboxylase. The tissue is pulverized, preferably after being frozen in liquid nitrogen to stop the chemical reactions within. The pulverized tissue is homogenized and extracted with a protein-extracting solution to form an extract containing the polypeptide along with many other polypeptides that are naturally present in the tissue. Preferably, the homogenizing and extracting steps are done at the same time. The protein extracting solution preferably comprises:

(1) a buffer such as Tris.HCl to stabilize the pH of the solution to near neutral (pH 7); (2) a reagent of complex divalent metal ions, such as ethylenediaminetetraacetic acid (EDTA); (3) a sulfhydryl reagent, such as 2-mercaptoethanol or dithiothrietol, to protect thiol groups; (4) protease inhibitors, such as phenylmethylsulfonyl fluoride (PMSF) or chymostatin; (5) anti-oxidation reagents, such as sodium bismetabisulfate and/or sodium thiosulfate and/or sodium sulfite; and (6) a protein denaturing agent, such as sodium dodecyl sulfate, urea, or guanidinium hydrochloride.

The extract is preferably separated from the remaining, solid part of the homogenate, preferably through the use of appropriate filters. Most preferably, any additional, suspended solid material is removed by centrifugation. The prepared extract or supernatant is then contacted with tetrameric avidin bound to a solid, insoluble support for a sufficient time and under appropriate conditions which will be known to those skilled in the art, given the teachings contained herein, to bind the polypeptides to the avidin. The solid support is preferably an agarose-based matrix contained in a chromatographic column. Any biotin-containing polypeptide in the extract, including the polypeptide of the invention, will bind tightly to the avidin. The bound polypeptides are removed from the avidin and recovered in isolated form through the application of standard techniques, given the teachings disclosed herein. Preferably, a strong denaturing agent is used to denature the avidin to release the biotin-containing polypeptide. Tetrameric streptavidin or an equivalent biotin-binding compound may be used instead of the avidin.

Generally, tissue containing the polypeptide of the invention will also contain other biotin-containing polypeptides. Accordingly, the desired polypeptide is recovered in purified form by separating the polypeptides removed from the avidin by molecular weight and recovering the 50 kilodalton polypeptide. For example, they are separated by gel electrophoresis, and the band of proteinaceous material at 50 kD is recovered by known techniques. If desired, the polypeptide can be further purified to remove unwanted, non-proteinaceous material (and any trace amounts of contaminating proteinaceous material) by the application of standard purification techniques.

It will be recognized by those skilled in the art that the polypeptide of the invention may be modified by known protein modification techniques. These include the techniques disclosed in U.S. Patent No. 4,302,386 issued November 24, 1981 to Stevens, incorporated herein by reference. Such modifications may enhance the activity of the antigen, or they may have no affect on such activity. For example, a few amino acid residues may be changed. Alternatively, the antigen of the invention may contain one or more amino acid sequences that are not necessary to its activity. Unwanted sequences can be removed by techniques well-known in the art. For example, the sequences can be removed via limited proteolytic digestion using enzymes such as trypsin or papain or related proteolytic enzymes.

Alternatively, polypeptides corresponding to functional parts of the polypeptide may be chemically synthesized by methods well-known in the art. These include the methods disclosed in U.S. Patent No. 4,290,944 issued September 22, 1981 to Goldberg, incorporated herein by reference.

Thus, the polypeptide of the invention includes a class of modified polypeptides, including synthetically derived polypeptides or fragments of the original polypeptide, having common elements of origin, structure, and mechanism of action that are within the scope of the present invention because they can be prepared by persons skilled in the art, once given the teachings disclosed herein. This includes polypeptides containing the deduced amino acid sequence coded for by the cDNA sequence of Figure 8, including fragments and variants of the sequence, that have catalytic activity for the carboxylation of acetyl-CoA to malonyl-CoA. Accordingly, all such modified polypeptides or peptide fragments are within the scope of the invention, provided that they have the specified catalytic activity.

The polypeptide of the invention is useful for several purposes. First, it will be useful for the scientific investigation of the important process of fatty acid biosynthesis in plants. The purified subunits can be used to define the structure of the biotin-addition site and thus lead to an understanding of how the biotin prosthetic group is added to the enzyme. Second, one or more of the subunits can be combined by non-covalent interactions (ionic, hydrogen bonding, or hydrophobic) to form compositions that catalyze the carboxylation of acetyl-CoA to malonyl-CoA and particularly plant acetyl-CoA to plant malonyl-CoA. In addition, since the naturally occurring acetyl-CoA carboxylase has a molecular weight of about 500 kD, the combination of ten of these subunits will result in the synthesis of purified plant acetyl-CoA carboxylase, which has been extremely difficult to purify in its complete and active form. This will permit the detailed study of this enzyme and a better understanding of fatty acid biosynthesis. The enzyme may also be useful in in vitro fatty acid synthesis. Third, the purified polypeptide of the invention is used to produce antibodies. The antibodies are used in an affinity chromatography column to purify the natural acetyl-CoA carboxylase in its active form and to screen a cDNA library to get the gene for the polypeptide.

The antibodies are used to obtain the purified native enzyme in active form as follows. Plant tissue containing acetyl-CoA carboxylase is pulverized and homogenized, forming a homogenate that contains the enzyme. The homogenate is extracted with a protein-extracting solution to form an extract, and the extract is separated from the remaining part of the homogenate, preferably, through the use of appropriate filters. Most preferably, any remaining suspended solid material is removed by centrifugation. The resulting prepared extract is then contacted with the antibodies for sufficient time and under appropriate conditions to bind the enzyme to the antibodies. The antibodies may be in solution, in which case the antibody-enzyme complexes need to be recovered. They may be recovered by contacting them with Protein A bound to a solid support. Alternatively, additional antibodies that bind to the complexes may be added to precipitate the complexes, whereupon they are easily removed from the solution. Preferably, the antibodies are bound to a solid, insoluble support. Preferably, the solid support is an agarose-base matrix, most preferably in a chromatography column. The enzyme is then eluted from the antibodies through the use of an appropriate eluting solution, thereby recovering it in purified form.

Given the instant polypeptide and the teachings disclosed herein, a person skilled in the art can obtain DNA coding for the polypeptide through the application of known molecular biology techniques that have been modified and applied in accordance with the discoveries and teachings described herein. Thus, the invention includes an isolated or substantially purified DNA sequence that codes for the biotin-containing polypeptide with a molecular weight of about 50 kilodaltons, which is a subunit of a plant acetyl-CoA carboxylase. Preferably, the DNA of the invention is a complementary DNA (cDNA). Most preferably, it contains the nucleotide sequence shown in Figure 8.

As used herein, the term "isolated" and variations thereof means that the DNA is in isolation from DNA encoding polypeptides normally accompanying this polypeptide. Thus, the DNA of the invention includes DNA encoding the polypeptide when that DNA has been cloned into a bacterial vector, such as a plasmid, or into a viral vector that may be harbored by a bacteriophage, provided that such clones are in isolation from clones that contain DNA encoding other polypeptides normally accompanying the instant polypeptide.

As used herein, the term "substantially pure" and variants thereof means that the DNA is substantially free of DNA and RNA that does not encode the polypeptide of the invention. That is, there is no more than about 1% of other DNA and RNA and preferably no more than about 0.2% of other DNA and RNA in any sample that contains the DNA encoding the polypeptide of the invention.

The polypeptide of the invention is used to make antibodies to it, by which cDNA coding for the polypeptide is obtained. The antibodies are prepared by standard techniques. Polyclonal or monoclonal antibodies may be used, although polyclonal are preferred.

The antibodies are used to screen a cDNA expression library. The library comprises cDNA from plant cells that express acetyl-CoA carboxylase that has been cloned into vectors. Different segments or fragments of the cDNA will have been operably and recoverably inserted into each vector so that each vector contains only one segment of the cDNA. The vectors may be plasmids or viruses. If necessary because of the type of library being used, the segments of cDNA will have been inserted into the vectors in a manner such that they will be expressed under appropriate conditions, i.e., in proper orientation and correct reading frame. Preferably, the library is a lambda gt11 library that contains cDNA derived from somatic embryos of carrot.

The library is screened by contacting the clones with the antibodies to the polypeptide to determine which clones bind to the antibodies. The clones are then isolated and recovered by known techniques. In the case of the lambda gt11 library, such isolation preferably comprises dilution of the identified plaques and rescreening with the antibodies until all of the plaques bind to the antibodies. The identity of the clone may be confirmed by standard techniques.

The protein expressed by the clone can be analyzed in order to confirm that the clone produces the polypeptide of the invention. For example, Western blot can be used in conjuction with the antibodies to the polypeptide. Alternatively, the cDNA clone can be analyzed by excising the cDNA with restriction enzymes, transforming a host cell that does not express the polypeptide with the cDNA to cause the cell to express it, and determining if the host cell expresses the polypeptide.

The inventors have used immunological screening of a cDNA expression library to obtain a clone, CC6, which is the 229 base pair cDNA sequence shown in Figure 8. Having such a sequence, the preferred method of obtaining the full cDNA sequence is to use this sequence as a probe to screen a reconstructed cDNA library that is enriched in full-length cDNA clones. The inventors will deposit clone CC6 with the American Type Culture Collection, 12301 Parklawn Drive, Rockville, Maryland 20852 USA for purposes of meeting the best mode requirement under U.S. patent law.

The preparation and screening of such a library involves techniques known in the art, modified in view of the teachings disclosed herein. Briefly, the following steps are involved. RNA is extracted and purified from plant tissues containing acetyl-CoA carboxylase, preferably from somatic embryos of carrot. Poly A RNA is isolated and purified from the total extracted RNA. The poly A RNA is utilized as a template for the synthesis of double-stranded, blunt-ended cDNA. To ensure that the cDNA library to be constructed will be enriched in long, full-length cDNA clones, the derived cDNA is size-fractionated by gel filtration chromatography. The fractions containing the larger cDNAs are pooled and used in subsequent cloning procedures. The cDNA is then cloned into a vector, preferably a lambda phage vector, such as lambda gt10, thereby producing the cDNA library. The library is screened with the cDNA clone of Figure 8 or appropriate fragments thereof to identify clones that hybridize to the probe. The positive recombinant clones are plaque purified. The cDNA is isolated from the positive clones and subjected to Southern blot analysis to confirm the identity of the clones and to determine the lengths of the cDNAs isolated. The longest cDNA is then subcloned into a plasmid vector for determination of its sequence.

With the cDNA encoding the polypeptide, a person skilled in the art can screen a genomic library to obtain the gene for the polypeptide, using standard techniques in conjunction with the teachings described herein. Briefly, genomic DNA is isolated from the organism of choice, in this case carrot. This DNA is hydrolyzed with a restriction enzyme whose recognition sequence occurs at random in the genome, such as Sau3A. The digestion is done under such conditions as to give rise to partially hydrolyzed DNA that is in the size range of 10-20Kbp. Such DNA is cloned into a lambda phage derived vector that carries a unique restriction site that will except Sau3A-ended DNA. An example of such a vector is Charon 34. The resulting library is screened with the appropriate cDNA as described above to isolate a full-length DNA.

Thus, the present invention encompasses an isolated or purified clone from a plant DNA library, which clone contains a DNA molecule encoding the polypeptide of the invention.
Preferably, the clone comprises an essentially pure culture of bacteriophage containing the cDNA. That is, none of the phage in the culture contains any cDNA other than cDNA encoding the polypeptide of the invention. Alternatively, the clone comprises an essentially pure culture of bacteria, such as E.coli, containing the cDNA of the invention inserted into a plasmid.

It will be recognized by persons skilled in the art that the cDNA sequence of the preferred polypeptide and the DNA comprising the gene may be modified by known techniques in view of the teachings disclosed herein. For example, different codons can be substituted that code for the same amino acid as the original codons. Alternatively, the substitute codons may code for a different amino acid that will not affect the activity or immunogenicity of the polypeptide or which may improve its activity or immunogenicity. For example, site-directed mutagenesis or other techniques to create single or multiple mutations, such as replacements, insertions, deletions, and transpositions, as described in Botstein and Shortle, "Strategies and Applications of In Vitro Mutagenesis," Science, 229:193-1210 (1985), which is incorporated herein by reference, can be employed. Since such modified DNA can be obtained by the application of known techniques to the teachings contained herein, such DNA is within the scope of the claimed invention.

Moreover, it will be recognized by those skilled in the art that the cDNA sequence (or fragments thereof) of the invention can be used to obtain other cDNA or DNA sequences that hybridize with it under conditions of high stringency. Such DNA includes any genomic DNA. Accordingly, the DNA of the invention includes DNA that shows at least 55 percent, preferably 60 percent, and most preferably 70 percent homology with the cDNA containing the sequence of Figure 8 or the genomic DNA from which such cDNA is derived, provided that such homologous DNA encodes an approximately 50 kD polypeptide that catalyzes the carboxylation of acetyl-CoA to form malonyl-CoA.

The DNA of the invention may be used in accordance with known techniques, appropriately modified in view of the teachings contained herein, to construct an expression vector, which is then used to transform an appropriate host cell for the expression and production of the polypeptide of the invention. Such techniques include those disclosed in U.S. Patent Nos. 4,440,859 issued April 3, 1984 to Rutter, et al., 4,530,901 issued July 23, 1985 to Weissman, 4,582,800 issued April 15, 1986 to Crowl, 4,677,063 issued June 30, 1987 to Mark, et al., 4,678,751 issued July 7, 1987 to Goeddel, 4,704,362 issued November 3, 1987 to Itakura, et al., 4,710,463 issued December 1, 1987 to Murray, 4,757,006 issued July 12, 1988 to Toole, Jr., et al., 4,766,075 issued August 23, 1988 to Goeddel, et al., and 4,810,648 issued March 7, 1989 to Stalker, all of which are incorporated herein by reference. Preferably, the host cell is a plant cell, which is transformed according to the techniques disclosed in Gordon-Kamm, et al., The Plant Cell 2:603 (1990), Paszkowski, et al., EMBO J. 3:2717 (1984), Rogers, et al., Methods Enzymol. 118:627 (1986), and U.S. Patent No. 4,801,540 issued January 31, 1989 to Hiatt, et al., all of which are incorporated herein by reference.

The DNA of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA, preferably the cDNA, is inserted into an expression vector, such as a plasmid, in proper orientation and correct reading frame for expression. If necessary, the DNA may be linked to the appropriate transcriptional and translational regulatory control nucleotide sequences recognized by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell. The preferred expression vector for use in the invention is the Ti or Ri plasmid or a derivative from Agrobacteria.

Host cells that have been transformed by the recombinant DNA of the invention are then cultured for an sufficient time and under appropriate conditions known to those skilled in the art in view of the teachings disclosed herein to permit the expression of the polypeptide, which can then be recovered. Alternatively, the recombinant DNA is used to transform plant cells from which whole plants can be recovered or to transform cells in existing plants.

Plant transformation can be accomplished by a variety of methods. For example, Agrobacteria spp., a natural plant pathogen, can be used to mediate transformation. These bacteria contain a plasmid (Ti or Ri plasmid); upon infection of a plant or plant cells by Agrobacteria, a region of the plasmid DNA (T-DNA) becomes stably integrated into the genome of the plant cells. A foreign gene of interest can be inserted into the T-DNA of such plasmids. Thus, when an Agrobacterium harboring such a modified plasmid is utilized to infect intact plants, plant cells, or plant cell protoplasts, the gene of interest is transferred into the plant genome. See Lichtenstein, C. P. and Draper, J. in DNA Cloning, Vol. 2, (D.M. Glover, ed.), IRL Press, Oxford, 1985, pp. 67, Rogers, et al., Methods Enzymol. 118:627 (1986), and the references cited therein, all of which are incorporated herein by reference. This procedure is particularly amenable for transformation of dicot plant species.

Alternately, and particularly for non-dicot plant species, foreign DNA can be introduced into the plant cells or protoplasts by disruption of the membrane by an osmotic or electric shock (Paszkowski, et al., EMBO J., 3:2717, (1984), Potrykus, Trends Biotech., 7:269 (1989), and references therein, all of which are incorporated herein by reference) or by "shooting" the DNA into the cells via a microprojectile bombardment (Gordon-Kamm et al., op. cit. and the references therein).

In the above techniques, typically, transformed cells are selected and plants are regenerated from single cells by standard tissue culture technologies.

The DNA for acetyl-CoA carboxylase has wide applications. It could be used to transform procaryotic and eucaryotic organisms to modify the genetic basis of the organism's metabolism. In addition, genetic engineering of the gene in oil seed crops, such as soybean, corn, sunflower, rape, and canola, offers the opportunity of altering oil seed quantity and quality. For example, the ratio of unsaturated to saturated fatty acids and/or the chain length of constituent fatty acids could be modified.

In addition, acetyl-CoA carboxylase in monocot plants, such as corn, wheat, barley, and sorghum, is inhibited by the aryloxyphenoxypropanoate and cyclohexanedione herbicides. However, acetyl-CoA carboxylase from dicots, such as carrot, is resistant to these compounds. Therefore, genetic engineering of the carrot acetyl-CoA carboxylase gene into monocot species would provide herbicide resistant plants.

Furthermore, the DNA could be used as a marker that would enable the selection of transformed monocot cells. Acetyl-CoA carboxylase from monocot species is susceptible to inhibition by the herbicides aryloxphenoxypropanoate and cyclohexanedione. Therefore, monocot plants, plant cells, and cell protoplast die upon their exposure to these herbicides. However, the acetyl-CoA carboxylase from dicot species, such as carrot, are resistant to these herbicides. Therefore, should the gene for the carrot acetyl-CoA carboxylase be used to transform monocot plant species, then the monocot plants, plant cells, and protoplasts that received the dicot gene would be able to survive exposure to these herbicides.

Another use of the acetyl-CoA carboxylase gene is to express it in its opposite orientation to produce antisense RNA. Such antisense RNA molecules would specifically reduce the synthesis of acetyl-CoA carboxylase, thus reducing the enzyme in plants. An alternative approach is to modify the acetyl-CoA carboxylase gene to produce an antisense RNA with a ribozyme inserted. Such a ribozyme would specifically hydrolyze the acetyl-CoA carboxylase in RNA, thus reducing the synthesis of the enzyme.

It is to be understood that application of the teachings of the present invention to a specific problem or environment will be within the capabilities of one having ordinary skill in the art in light of the teachings contained herein. Examples of the products of the present invention and processes for their production appear in the following examples.

EXAMPLE 1

Purification of Polypeptide and Isolation of cDNA

This Example reports the purification of a 50Kd biotin-containing polypeptide from carrot that represents the biotin subunit of one form of acetyl-CoA carboxylase. A cDNA clone coding for this polypeptide has been isolated and has been utilized to investigate the mRNA and gene(s) coding for this acetyl-CoA carboxylase.

Materials and Methods

Plant materials and tissue culture. Seeds of Daucus carota var. Danver were germinated in a sterile soil mix and plants were grown in a greenhouse at 18-22°C under ambient illumination. Carrot cultures were initiated and maintained as previously described (1, 2). Briefly, embryogenic carrot cultures were initiated from the secondary phloem of the roots on a solid Murashige and Skoog medium (3) containing 0.5 mg/l of 2,4-D. Embryogenic callus was selected and inoculated into an identical liquid medium but without agar. Such callus suspension cultures were maintained by transferring into fresh medium at monthly intervals. Somatic embryogenesis was induced by transferring callus into an identical liquid medium lacking the auxin 2,4-D. Two weeks after transfer mature embryos were collected on a stainless steel screen of 1910um mesh size.

Purification of biotin proteins. Somatic embryos (500g) were frozen in a mortar with liquid nitrogen and while frozen they were pulverized with a pestle to a fine powder. After the addition of 500ml of extraction buffer (50mM Tris-HCl, pH 7.0, 10mM DTT, 5mM EDTA, 5mM sodium bismetabisulphite, 2mM benzamidine, 2% (w/v) SDS, 5% (v/v) glycerol, 0.05% (v/v) Triton X-100 and 100 µg/ml PMSF), homogenization was carried out on ice as the slurry melted. Once homogenization was completed the extract was quickly heated in a boiling water bath and kept at 100°C for 20-30 minutes. The extract was filtered thru 2 layers of cheesecloth, one layer of Miracloth and the filtrate was clarified by centrifugation at 30,000g for 30 minutes. Biotin proteins were specifically collected from the supernatant with immobilized tetrameric avidin. To each 100ml aliquot of supernatant 2.5 ml of packed cross-linked agarose-avidin was added and the mixture stirred for at least 2 hours. The agarose-avidin was collected by centrifugation (1,600g for 10 min) and washed three times with 2% (w/v) SDS, 5% (v/v) glycerol, 20mM Tris-HCl, pH 7.0, 2mM DTT and 1mM EDTA (Wash Buffer). Biotin proteins were eluted from the Agarose-avidin by heating the suspension in a boiling waterbath and filtering it thru a glass-fritted column. The resin was washed with one volume of wash buffer heated to between 85° and 95°C. The eluted protein was concentrated by ultrafiltration with Centricon 10 membranes.

Antisera. The purified biotin proteins were subjected to preparative SDS-PAGE (4) in 1.5mm thick slabs of 12% acrylamide. Following staining with Coomassie Brilliant Blue and destaining, the 50kD biotin protein band was excised from the slab, minced and pulverized with a mortar and pestle and emulsified with Freund's Complete Adjuvant. Emulsion containing approximately 300µg of protein was injected subcutaneously at multiple sites on the back of New Zealand White female rabbits. A month following the initial immunization and at two week intervals thereafter, these rabbits were challenged with muscular injections of 150µg-200µg of the 50kD biotin protein. One week following each injection, 2-3ml of blood was withdrawn from the ear of each rabbit, allowed to coagulate and the antisera collected. The titre of each antisera was monitored by an ELISA method (5, 6).

Isolation of cDNA encoding 50kD biotin protein. A cDNA library constructed from poly A RNA isolated from developing somatic embryos of carrot was constructed in the vector lambda gt11 (kindly provided by Dr. Terry Thomas, Department of Biology, Texas A&M University). 300,000 recombinant phage plaques were immunologically screened basically as described by Huynh (7). Recombinant bacteriophage producing LacZ fusion protein immunologically recognized by the antisera directed against the 50kD biotin protein were plaque purified.

Analysis of protein, DNA, and RNA. Following SDS-PAGE, proteins were electrophoretically transferred to a Nitrocellulose membrane (8). The 50kD biotin protein was specifically detected by a immunological Western procedure using $^{125}$I-labelled Protein A to visualize the antigen-antibody complex. $^{125}$I-labelled streptavidin was used to specifically visualize biotin-containing proteins (9).

DNA was isolated from carrot tissues by the method of Dellaporta et al. (10). Plasmid DNA was isolated by a rapid, small-scale (11) or a large-scale (12) procedure. Lambda bacteriophage DNA was isolated from small-scale and large-scale cultures. Isolated DNA was digested with restriction endonucleases as recommended by the supplier and digestion products were fractionated by electrophoresis in agarose gels buffered with TAE (13). Restriction endonuclease digested DNA fragments were purified from agarose slabs by the method of Vogelstein and Gillespie (14). DNA fragments were radioactively labelled with (alpha-$^{32}$P)dCTP to a specific radioactivity of approximately $1 \times 10^9$ cpm/µg of DNA, by either nick translation (15) or random-primer extension (16). Electrophoretically fractionated DNA was alkali denatured neutralized and transferred to Magnagraph Nylon 66 membrane by capillary action (17) using 25mM sodium phosphate buffer, pH 6.5. Immobilized DNA was subjected to prehybridization and hybridization at 60°C in a solution of 10% (w/v) dextran sulphate, 5X SSC, 1X Denhardt's, 50mM Tris-HCl, pH 8.0, 0.2% (w/v) SDS, 10mM EDTA and 100µg/ml single-stranded, sheared salmon sperm DNA.

RNA was extracted form carrot tissues as described by Berry et al. (18). Following denaturation with 50% (v/v) formamide, 6% (v/v) formaldehyde, RNA was fractionated by electrophoresis in agarose gels containing 6% (v/v) formaldehyde (19), buffered with 20mM sodium phosphate buffer, pH 7.7. Fractionated RNA was transferred to a Nylon66 membrane by capillary action (20) using 25mM sodium phosphate buffer, pH 6.5. Immobilized RNA was subjected to prehybridization and hybridization at 42°C in a solution of 50% (v/v) formamide, 5X SSPE, 5X Denhardt's, O.1% (w/v) SDS and 100µg/ml single-stranded, sheared salmon sperm DNA.

Results

Purification of biotin-containing polypeptides and antibody production. The purification of sufficient quantities of acetyl-CoA carboxylase from plants for structural or biochemical analysis has been problematic. Even the use of affinity chromatography with immobilized monomeric avidin which has been successfully utilized for the purification of many biotin enzymes has not yielded satisfactory results with plant acetyl-CoA carboxylases. We therefore chose to purify biotin-containing polypeptides independent of their enzymatic function and use the purified protein as an antigen to obtain antisera that could subsequently be used to identify and the characterize the enzyme that each polypeptide represents.

Developing somatic embryos, between 2 and 4 weeks after induction, were utilized for the purification of the biotin-containing polypeptides. Proteins were extracted from embryos with a buffer containing 2% SDS and the extract was immediately heated in a boiling waterbath for a period of 20-30 minutes. This extraction procedure denatured all the proteins in the extract. To collect the biotin-containing polypeptides, the extract was incubated with Agarose-tetrameric avidin at room temperature for a period of at least 2 hours. The non-biotin-containing proteins were washed from the matrix by centrifugation and the biotin-containing proteins were eluted from the matrix by placing it in a boiling water bath and washing it with hot 2% SDS.

The eluted biotin-containing proteins were subjected to SDS-PAGE and Western analysis to determine the purity of the preparation. Coomassie Blue staining of the gel indicated the presence of two predominant polypeptides, with relative molecular weights of 70,000 and 50,000. See Figure 1. A Western blot of an identical gel incubated with $^{125}$I-streptavidin, indicated that these two polypeptides contained biotin and that this preparation also contained biotin-containing polypeptides of 220Kd, 120Kd, 36Kd and 32Kd, which accounts for all the biotin-containing polypeptides that are present in this tissue. From about 500g of fresh tissue, we estimate a yield of approximately 10mg of pure biotin-containing proteins.

To elucidate the enzymatic and subsequently the physiological functions of these biotin-containing proteins, we needed to obtain these proteins in their native state. The approach we undertook was to obtain antibodies that react with each of these biotin-containing proteins and utilize these antibodies to investigate the functions of each of the proteins. Rabbits were immunized with gel purified 50Kd biotin-containing protein as outlined in the Methods. Serum obtained from such rabbits was utilized in Western analysis of carrot embryo extract to evaluate the specificity of the antibodies.

A carrot embryo extract that contained the six biotin-containing polypeptides found in plants, as visualized with $^{125}$I-streptavidin, was also subject to Western analysis with the antiserum obtained from rabbits that were immunized with the 50Kd biotin-containing protein. Such analyses showed that this antiserum contained antibodies that react solely and specifically with the 50Kd biotin-containing polypeptide. See Figure 2. Therefore, this protein is immunologically distinct from all the other biotin-containing proteins that are found in this extract. Furthermore, since the antiserum specifically reacted only with the 50Kd biotin-containing protein, it could be utilized as a reagent to investigate the specific function of this protein.

Enzymatic function of the 50Kd biotin-containing polypeptide. The antiserum that reacts with the 50Kd biotin-containing polypeptide was utilized to identify the enzymatic function of this polypeptide. Aliquots of an extract prepared from carrot embryos were incubated with increasing quantities of preimmune serum or serum obtained from rabbits immunized with the 50Kd biotin-containing polypeptide. The antigen-antibody complexes that were formed were collected by binding to Protein A attached to Agarose beads. These complexes were removed by centrifugation and the supernatants were assayed for acetyl-CoA carboxylase, propionyl-CoA carboxylase and methylcrotonyl-CoA carboxylase activities. See Figure 3.

Preimmune serum had little effect on all these activities in this experiment. The serum obtained from rabbits immunized with the 50Kd biotin-containing polypeptide had an effect on acetyl-CoA carboxylase activity. Increasing amounts of antiserum in this experiment resulted in the removal of up to half the acetyl-CoA carboxylase activity that was present in the extract. Methylcrotonyl-CoA carboxylase activity was unaffected by the antiserum. The small amount of propionyl-CoA carboxylase activity that was removed by the antiserum was probably due to the fact that acetyl-CoA carboxylase can also catalyze the carboxylation of propionyl-CoA. These results therefore lead us to conclude that the 50Kd biotin-containing polypeptide represents a subunit of an acetyl-CoA carboxylase.

Isolation of a cDNA clone of acetyl-CoA carboxylase. An expression cDNA library constructed in the lambda based vector gt11, prepared from poly A RNA isolated from developing somatic embryos of carrot was kindly provided by Dr. T. Thomas. This library was screened for recombinant phage which produced a LacZ fusion protein that contained epitopes recognized by the antibodies to the 50Kd biotin-containing polypeptide. Screening of 200,000 recombinant phage resulted in the isolation of five cDNA clones putatively identified as coding for acetyl-CoA carboxylase. See Figure 4.

Verification of the identity of these clones was achieved by carrying out Western analysis on lysates prepared from lysogens of recombinant phage that were putatively identified as coding for the 50Kd biotin-containing polypeptide. A lysogen carrying one of the recombinant phage (CC6) produced a LacZ fusion protein of 128Kd that contained unique epitopes recognized by the antibodies to the 50Kd biotin-containing polypeptide that are absent from the LacZ protein. See Figure 5. This LaccZ fusion protein coded by CC6 was approximately 10Kd larger than the wild-type LacZ protein produced by a lysogen of lambda gt11; this difference in size between the fusion recombinant protein and LacZ is consistent with the 229bp size of the insert in the CC6 recombinant phage.

Acetyl-CoA carboxylase transcript. To identify the size of the mRNA that codes for the subunit of this acetyl-CoA carboxylase, and to identify the developmental stages at which this gene is active, RNA isolated from a variety of carrot organs was fractionated by electrophoresis in formaldehyde-containing agarose gels. Following Northern transfer of the RNA to a membrane, the mRNA for acetyl-CoA carboxylase was detected by hybridization with the 229bp insert from the CC6 clone. A single RNA of about 2.0Kb was detected in RNA samples isolated from developing somatic embryos of carrot. See Figure 6. The relative abundance of this RNA was unchanged as embryos developed from the globular to the torpedo stage. This RNA was detected at approximately the same abundance in roots of carrot; however, it could not be detected in RNA isolated from leaves.

Acetyl-CoA carboxylase gene organization. To gain an insight as to the number of genes in the carrot genome that code for the subunit of this acetyl-CoA carboxylase, total DNA was isolated from carrot leaves. This DNA was hydrolyzed with restriction endonucleases, and fractionated by electrophoresis in an agarose gel. Following Southern transfer, the acetyl-CoA carboxylase genes were detected by hybridization. The insert from the CC6 clone hybridized to at least six DNA fragments generated by digestion with EcoRI or HindII. See Figure 7. Should each of these fragments represent independent loci in the genome, these results lead us to conclude that this acetyl-CoA carboxylase is coded by a small gene family with possibly up to six members per diploid genome.

Sequence of CC6. To determine the DNA sequence of the cDNA clone, CC6, the insert was subcloned into the Eco RI site of the plasmid pUC19. DNA sequencing was carried out by the method of Sanger, et al. (21) using double stranded DNA as template. The sequence is shown in Figure 8. Comparison of this sequence to the EMBL Gene databank did not reveal any significant similarity between this sequence and any sequences in the databank.

Because the sequence is relatively short, the translational reading frame could not be identified with certainty. However, this clone will have great utility in detecting the mRNA and genes of acetyl-CoA carboxylase. Furthermore, efforts are underway to utilize this sequence as a probe to isolate a full-length cDNA that can be used to determine the complete sequence of the acetyl-CoA carboxylase subunit.

Discussion

The enzymatic and metabolic functions, structures, and mechanisms of regulation of biotin enzymes of plants are poorly understood. Developing somatic embryos of carrot offer an interesting system to study these aspects of biotin enzymes of plants, since they accumulate these proteins to relatively high levels and this accumulation is under developmental control. Like most plants examined, carrots contain a number of biotin proteins. Western analyses of protein extracts from developing somatic embryos with $^{125}$I-labelled streptavidin detects at least six biotin-containing proteins. Since acetyl-CoA carboxylase was thought to be the only biotin enzyme in plants, the multiplicity in biotin proteins was suggested to be due to the presence of isoenzymes of acetyl-CoA carboxylase. However, the recent discovery of three additional biotin enzymes in plants, pyruvate carboxylase, propionly-CoA carboxylase, and 3-methylcrotonyl-CoA carboxylase, offers an additional explanation for the function of the six biotin proteins found in carrot.

In order to address the questions of the functions, structures, and regulation of these enzymes, we undertook the purification of these proteins and subsequent isolation of cDNA clones representing these enzymes. Purification of these proteins is greatly facilitated, since the covalent attachment of the biotin prosthetic group allows the use of avidin for their affinity purification. Initial attempts to purify plant acetyl-CoA carboxylases and other biotin-containing protein in their native state by the use of affinity matrices composed of monomeric avidin proved disappointing in that the yields of purified protein were very low.

To overcome this problem, we decided to first purify the biotin-containing proteins independent of their enzymatic function, utilizing tetrametic avidin as the affinity matrix. Since the affinity for biotin by avidin is extremely high ($K_D=10^{-15}$), the elution of the biotin-containing proteins from the affinity matrix could only be carried out by denaturation of the immobilized avidin with boiling 2% SDS. Therefore, since the purified proteins were obtained in a denatured state, we decided to make our initial extracts with boiling 2% SDS. This denaturation also improved the yield of purified biotin-containing proteins, possibly because the denaturation of these protein exposes the biotin prosthetic group, thus making it more available for binding to the immobilized avidin.

This strategy yielded sufficient quantities of pure protein for the generation of antibodies that could be utilized for the identification of their enzymatic function. The antibodies generated to the 50Kd biotin-containing polypeptide reacted solely with that protein. Therefore, this protein is immunologically distinct from all the other biotin-containing proteins that are found in the carrot embryo extracts.

The antibodies to the 50Kd biotin-containing polypeptide, in association with Protein A, removed acetyl-CoA carboxylase activity from extracts that contained the three biotin enzymes, acetyl-CoA carboxylase, propionyl-CoA carboxylase, and methylcrotonyl-CoA carboxylase, thus establishing that this polypeptide is a subunit of an acetyl-CoA carboxylase. This finding clarifies to some extent the confusion as to the size of the biotin-containing subunit of the plant acetyl-CoA carboxylase. Various workers have reported biotin-containing subunits of acetyl-CoA carboxylase varying in size from 220Kd to 21Kd. The suggestion that there may be more than one form of acetyl-CoA carboxylase has been made, and indeed the results reported here substantiate this hypothesis. The antibodies against the 50Kd biotin-containing polypeptide remove only half the acetyl-CoA carboxylase activity that is present in the extract. The fraction that remains in the extract is presumably immunologically distinct from the 50Kd subunit acetyl-CoA carboxylase, thus representing a different isoenzyme of acetyl-CoA carboxylase.

The presence of at least two forms of acetyl-CoA carboxylase provides plants with the ability to differentially regulate the synthesis of malonyl-CoA, which is an intermediate in the synthesis of a variety of phytochemicals, including fatty acids, very-long chain fatty acids, flavonoids, malonyl derivatives of D-amino acids and amino cyclopropionate carboxylic acid, and malonic acid. The metabolic function of the acetyl-CoA carboxylase with a 50Kd biotin-containing subunit has yet to be determined.

A cDNA clone coding for the 50Kd biotin-containing polypeptide was isolated by immunologically screening a cDNA expression library. Five putative clones were isolated. The authenticity of these cones were confirmed by Western analysis of the LacZ fusion proteins that were synthesized by lysogens of the recombinant phage. The longest cDNA insert is 229bp in length and is obviously a partial cDNA. Although the sequence of this clone cannot be used to deduce the amino acid sequence of acetyl-CoA carboxylase, it is an adequate probe for the detection of the mRNA and genes that codes for the 50K biotin-containing polypeptide.

The mRNA coding for the 50Kd biotin-containing polypeptide is approximately 2000bp in length. Such a length of sequence has ample capacity to code for this polypeptide. Interestingly, the mRNA does not accumulate in mature leaves; however, it accumulates to relatively high levels in developing somatic embryos and roots of carrot. The organ-specific accumulation of this mRNA may help in elucidating the metabolic function of this isoform of acetyl-CoA carboxylase.

Southern analysis of DNA isolated from carrots indicates that this acetyl-CoA carboxylase is coded by a small gene family, with possibly six individual members to the family. To determine whether all these genes are active at all developmental times will require further experimentation.

The molecular cloning of a sequence that codes for acetyl-CoA carboxylase will enable us to investigate the structure of this important enzyme. Furthermore, it will be possible to investigate the molecular genetic mechanisms that regulate this enzyme and thus control its metabolic function.

## EXAMPLE 2

### Cloning a Full-Length cDNA of Acetyl-CoA Carboxylase

This Example shows how to construct a cDNA library that is enriched in full-length cDNA clones and to screen this library with antibodies to the polypeptide of the invention. The steps are identified below.

1. Isolate poly A RNA: RNA will be extracted and purified from somatic embryos of carrot utilizing the procedure outlined in Berry et al. (22). Basically, the procedure consists of extracting the tissue with a buffer consisting of 50mM Tris-HCl (pH 8.0), 10mM EDTA, and 2% SDS. Following two extractions with phenol:chloroform:isoamyl alcohol (25:24:1), the nucleic acids in the aqueous phase are precipitated with 0.3M sodium acetate at 70% ethanol. The precipitated nucleic acids are collected by centrifugation, and the pellet is dissolved in 5 ml of water. To selectively precipitate RNA, an equal volume of 4M LiCl is added, and the mixture incubated on ice for 4 hours. The precipitated RNA is collected by centrifugation, and the pellet is dissolved in 0.5 ml of water. The RNA is reprecipitated at 0.3M sodium acetate and 70% ethanol. The RNA is collected by centrifugation and the pellet is dissolved in minimal volume of water.

Poly A RNA is purified from total RNA preparations by affinity chromatography with an oligo(dT)-cellulose column (Aviv and Leder) (23). Total RNA will be loaded onto an approximately 1 ml of a packed oligo(dT)-cellulose column equilibrated with a buffer containing 0.5M NaCl. Unbound RNA will be washed off with 5-10 column volumes of this buffer, and poly A RNA will be eluted with a buffer lacking any salt. The Poly A RNA will be precipitated at 0.3M sodium acetate and 70% ethanol, collected by centrifugation, and dissolved in a minimal volume of water.

2. Synthesis of double stranded cDNA. Poly A RNA will be utilized as template for the synthesis of double-stranded, blunt ended cDNA for cloning. The procedure that will be used was described by Gubler and Hoffman (24) and is the basis of many commercial kits available for the synthesis of cDNA. The poly A RNA template will be primed with oligo(dT) and will be copied as cDNA with reverse transcriptase. Second strand synthesis will be achieved by the combined use of RNase H and Escherichia coli DNA polymerase 1. To ensure that the resultant cDNA is blunt-ended, it will be treated with T4 DNA polymerase.

3. Size selection of cDNA. To ensure that the cDNA library that will be constructed is enriched in long, full-length cDNA clones, the cDNA derived from the above series of reactions will be size fractionated by gel filtration chromatography (Koike, et al.) (25). The cDNA will be chromatographed through a column of Sephacryl S200, and the fractions containing cDNA larger than 500-700bp will be pooled and used in subsequent cloning procedures.

4. Cloning the cDNA. The cDNA will be cloned into the unique Eco RI site of the lambda vector gt11. To achieve this, the ends of blunt-ended, double-stranded cDNA will be converted to cohesive Eco RI overhangs. This will be achieved by ligating with T4 DNA ligase a dephosphorylated Eco RI adaptor to the cDNA (26, 27). The Eco RI adaptors are blunt at one end and carry a preformed Eco RI cohesive end at the other. The Eco RI-adapted DNA is next purified from unreacted adaptors by gel filtration (Haymerle, et al. 1986) (28). The 5′ ends of the adapted DNA are then phosphorylated with T4 polynucleotide kinase and ligated with T4 DNA ligase to dephosphorylated lambda gt11 arms (Huynh, et al.) (29). The recombinant phage DNA will be packaged into phage particles using an in vitro packaging system (Collins and Hohn) (30).

5. Immunological Screening of the cDNA library for a cDNA coding for acetyl-CoA carboxylase. The resulting library of recombinant phage will be grown on the E. coli host Y1090. To identify and isolate the specific recombinant clone that codes for acetyl-CoA carboxylase, the library will be screened with the antibody to acetyl-CoA carboxylase (29, 31). Approximately 200,000 recombinant phage will be used to infect the E. coli host Y1090, and the infected cells will be plated in six 150cm petri plates of solid NZCYM media and grown at 42°C for 3-4 hours. The plates are then cooled to 37°C and overlayed with nitrocellulose filters that had previously been soaked with IPTG. The plates are further incubated at 37°C to induce the synthesis of the recombinant LacZ fusion protein.

The nitrocellulose filters with an imprint of the phage are subsequently incubated sequentially with antibodies to acetyl-CoA carboxylase and $^{125}$I-labeled Protein A. The position of the recombinant phage producing LacZ-acetyl-CoA carboxylase fusion protein is determined by exposing the filters to X-ray film. The area of the plate containing the recombinant phage coding for acetyl-CoA carboxylase will be excised from the plate and the phage eluted into an appropriate buffer. This process will be repeated at least three times resulting in the plaque purification of the appropriate recombinant phage.

6. Confirmation of the identity of the recombinant bacteriophage clone. To verify the identity of the putatively identified full-length cDNA clone, a lysogen of the recombinant phase is produced in the E. coli host Y1089 as described (29, 31). A protein lysate from such a lysogen is subjected to Western analysis with the antibodies to acetyl-CoA carboxylase to confirm that the isolated recombinant phage does in fact produce a LacZ-acetyl-CoA carboxylase fusion protein and thus the isolated cDNA clone codes for acetyl-CoA carboxylase.

EXAMPLE 3

Cloning a Full-Length cDNA of Acetyl-CoA Carboxylase

This Example shows how to construct a cDNA library that is enriched in full-length cDNA clones and to screen this library with CC6. Steps 1-3 are the same as in Example 2. Step 4 is the same as in Example 2, except the vector is lambda gt10 instead of lambda gt11. Steps 5 and 6 are shown below.

5. Screen the cDNA library for a full-length cDNA coding for acetyl-CoA carboxylase. The resulting library of recombinant phage will be propagated on the E. coli host NM514. This host allows for the genetic selection of recombinant lambda gt10 phage (Scherer, et al.) (32). To identify and isolate the specific recombinant clone that codes for acetyl-CoA carboxylase, the library will be screened by in situ hybridization of bacteriophage plaques with $^{32}$P-labeled CC6 DNA (Benton and Davis) (33). Approximately 200,000 recombinant phage will be used to infect the E. coli host NM514, and the infected cells will be plated in six 150cm petri plates of solid NZCYM media. An imprint of the phage DNA will be transferred nitrocellulose filters by capillary action. The position of the recombinant phage coding for acetyl-CoA carboxylase will be determined by hybridizing the filter with $^{32}$P-labeled CC6 DNA. The area of the plate containing the recombinant phage coding for acetyl-CoA carboxylase will be excised from the plate and the phage eluted into an appropriate buffer. This process will be repeated at least three times resulting in the plaque purification of the appropriate recombinant phage.

6. Confirmation of the identity of the recombinant bacteriophage clone. To verify the identity of the putatively identified full-length cDNA clone, DNA will be isolated from the purified recombinant clone (Sambrook, et al.) (31).

It will be apparent to those skilled in the art that various modifications and variations can be made to the products and processes of the present invention. Thus, it is intended that the present invention covers such modifications and variations, provided they come within the scope of the appended claims and their equivalents.

REFERENCES

1. Wurtele, E.S., Keller, G.L., Nikolau, B.J. and Ulrich, T.H. (1988) J. Plant Physiol. 132:683-689.

2. Keller, G.L., Nikolau, B.J., Ulrich, T.H. and Wurtele, E.S. (1988) Plant Physiol. 86:451-456.

3. Murashige, T. and Skoog, F. (1962) Physiol. Plant. 15:473-497.

4. Laemmli, U.K. (1970) Nature (London) 227: 680-685.

5. Engvall, E. and Perlmann, P. (1972) J. Immunol. 109: 129-135.

6. Voller, A., Bidwell, D.E. and Bartlett, A. (1976) Bull. W.H.O. 53:55-65.

7. Huynh, T.V., Young, R.A. and Davis, R.W. (1985) in DNA Cloning: A Practical Approach, Vol. 1, (D.M. Glover, ed.) IRL Press, Washington, D.C., pp. 49-78.

8. Towbin, H., Staehelin, T. and Gordon, J. (1979) Proc. Natl. Acad. Sci. U.S.A. 76:4350-4354.

9. Nikolau, B.J., Wurtele, E.S. and Stumpf, P.K. (1985) Anal. Biochem. 149:448-453.

10. Dellaporta, S.L., Wood, J. and Hicks, J.B. (1983) Plant Mol. Biol. Rep. 1:19-21.

11. Holmes, D.D. and Quigley, A. (1981) Anal. Biochem. 114:193.

12. Birnboim, H.C. and Doly, J. (1979) Nuc. Acid Res. 7, 1513.

13. Maniatis, T., Fritsch, E.F. and Sambrook, J. (1982) In Molecular Cloning: A Laboratory Manual. Cold Spring Harbour, N.Y.

14. Vogelstein, B. and Gillespie, D. (1979) Proc. Natl. Acad. Sci. U.S.A. 76:615-619.

15. Rigby, P.W.J., Dieckmann, M., Rhodes, C. and Berg, P. (1977) J. Mol. Biol. 113:237.

16. Gillespie, D. and Vogelstein, B. (1983) Anal. Biochem. 132:6-13.

17. Southern, E.M. (1975) J. Mol. Biol. 98:503.

18. Berry, J.O., Nikolau, B.J., Carr, J.P. and Klessig, D.F. (1985) Mol. Cell. Biol. 5:2238-2246.

19. Sambrook, J., Fritsch, E.F. and Maniatis, T. (1989) DNA Cloning: A Laboratory Manual, Cold Spring Harbor Laboratory Press.

20. Southern, E.M. (1975) J. Mol. Biol. 98:503.

21. Sanger, F., Miklan, S. and Coulson, A.R. (1977) Proc. Natl. Acad. Sci. U.S.A. 74:5463.

22. Berry, J.O., Nikolau, B.J., Carr, J.P. and Klessig, D.F. (1985) Mol. Cell Biol. 5:2238-2246.

23. Aviv, H. and Leder, P. (1972) Proc. Natl. Acad. Sci. USA, 69:1408.

24. Gubler, U. and Hoffman, B.J. (1983) Gene 25:263.

25. Koike, S., Sakai, M. and Muramatsu, M. (1987) Nucl. Acid Res., 15:2499.

26. Sartoris, S., Cohen, E.B. and Lee, J.S. (1987) Gene, 56:301.

27. Stover, C.K., Vodkin, M.H. and Oaks, E.V. (1987) Anal. Biochem., 163:398.

28. Haymerle, H., et al. (1986) Nucl. Acid. Res., 14:8615.

29. Huynh, T.V., Young, R.A. and Davis, R.W. (1985) in DNA Cloning, Vol. 1, (D.M. Glover, ed.), IRL Press, Washington, D.C. pp. 49.

30. Collins, J. and Hohn, B., Proc. Natl. Acad. Sci. USA, 71:2442.

31. Sambrook, J., Fritsch, E.F. and Maniatis, T. (1989) DNA Cloning: A Laboratory Manual, Cold Spring Harbor Laboratory Press.

32. Scherer, G., et al. (1981), Dev. Biol. 86:438.

33. Benton, W.D. and Davis, R.W. (1977) Science, 196:180.

Claims

1. A purified biotin-containing polypeptide with a molecular weight of about 50 kilodaltons, wherein said polypeptide is a subunit of plant acetyl-CoA carboxylase.

2. The polypeptide of claim 1 wherein said polypeptide contains amino acids encoded by the cDNA sequence shown in Figure 8.

3. The polypeptide of claim 1 wherein said polypeptide catalyzes the carboxylation of acetyl-CoA to form malonyl-CoA.

4. A polypeptide derived from the polypeptide of claim 3, wherein said polypeptide catalyzes the carboxylation of acetyl-CoA to form malonyl-CoA.

5. A method of obtaining biotin-containing polypeptides in isolation from non-biotin-containing polypeptides from plants comprising the steps of: extracting plant tissue containing said polypeptides with a protein-extracting solution to form an extract containing said polypeptides;

contacting said extract with tetrameric avidin or tetrameric streptavidin bound to a solid support for a sufficient time and under appropriate conditions to bind said polypeptides to said avidin or streptavidin; and

removing said polypeptides from said avidin or streptavidin, thereby recovering said polypeptides in isolated form.

6. The method of claim 5 wherein said tetrameric avidin to streptavidin bound to a solid support is in a chromatographic column.

7. The method of claim 5 further comprising the step of separating all recovered biotin-containing polypeptides by molecular weight, thereby recovering said polypeptides in purified form.

8. A method of obtaining the polypeptide of claim 1 comprising the steps of:

freezing plant tissue containing acetyl-CoA carboxylase by contacting said tissue with liquid nitrogen;

pulverizing said tissue while in contact with said liquid nitrogen;

homogenizing said pulverized tissue to form a homogenate containing said polypeptide;

extracting said homogenate with a protein-extracting solution to form an extract containing said polypeptide;

separating said extract from the remaining part of said homogenate;

contacting said separated extract with tetrameric avidin or tetrameric streptavidin bound to a solid support for a sufficient time and under appropriate conditions to bind said polypeptide to said avidin or streptavidin;

removing said polypeptide from said avidin or streptavidin;

separating all recovered biotin-containing polypeptides by molecular weight; and

recovering in purified form the polypeptide with a molecular weight of about 50kD.

9. A method of obtaining plant acetyl-CoA carboxylase in purified form comprising the steps of:

extracting plant tissue containing acetyl-CoA carboxylase with a protein-extracting solution to form an extract containing said plant acetyl-CoA carboxylase;

contacting said extract with antibodies to the polypeptide of claim 1 for a sufficient time and under appropriate conditions to bind said acetyl-CoA carboxylase to said antibodies;

separating said acetyl-CoA carboxylase bound to said antibodies from said extract; and

removing said acetyl-CoA carboxylase from said antibodies, thereby recovering said acetyl-CoA carboxylase in purified form.

10. The method of claim 9 wherein said antibodies are bound to a solid support.

11. The method of claim 9 wherein said separating step comprises contacting said extract containing the complex comprising acetyl-CoA carboxylase bound to said antibodies with an antibody-binding compound bound to a solid support for a sufficient time and under appropriate conditions to bind said complex to said antibody-binding compound.

12. A method for obtaining an isolated or substantially purified cDNA sequence coding for a biotin-containing polypeptide with a molecular weight of about 50 kilodaltons, wherein said polypeptide is a subunit of plant acetyl-CoA carboxylase, comprising the steps of:

preparing antibodies to said polypeptide;

screening a cDNA expression library with said antibodies to identify a clone producing said polypeptide, wherein said library comprises clones of vectors into which different sequences of plant cDNA have been operably and recoverably inserted, each of said vectors containing only one sequence of said cDNA; and

isolating said clone.

13. The method of claim 12 further comprising the step of recovering said cDNA sequence from said clone.

14. The method of claim 13 further comprising the step of evaluating said cDNA sequence to confirm that it codes for said polypeptide.

15. The said purified cDNA sequence obtainable by the method of claim 13.

16. An isolated or substantially purified DNA sequence that codes for the polypeptide of claim 1.

17. The DNA sequence of claim 16 wherein said DNA is cDNA.

18. The DNA sequence of claim 17 wherein said cDNA sequence contains the nucleotide sequence shown in Figure 8.

19. An isolated or substantially purified DNA sequence derived from the DNA sequence of claim 18 by single or multiple mutations.

20. A DNA sequence that hybridizes with the cDNA sequence of claim 18 under conditions of high stringency.

**21.** A recombinant DNA sequence comprising the DNA sequence of claim 16 operably linked to appropriate regulatory control nucleic sequences that are capable of effecting the expression of said DNA sequence in transformed host cells.

**22.** An expression vector for expressing DNA that codes for the protein of claim 1 in a compatible host comprising an expression vector capable of transforming a procaryotic or eucaryotic cell and the DNA of claim 16 inserted into said vector in proper orientation and correct reading frame for expression.

**23.** A host cell transformed with the recombinant DNA sequence of claim 21.

**24.** The host cell of claim 23 wherein said cell is a plant cell.

**25.** The host cell of claim 24 wherein said plant cell is a monocot cell.

**26.** The recombinant protein obtainable from the transformed cell of claim 23.

**27.** A method for producing the protein of claim 1 comprising the steps of:
culturing host cells transformed by a recombinant DNA sequence comprising a DNA sequence that codes for the protein of claim 1 operably linked to appropriate regulatory control nucleic acid sequences that are capable of effecting the expression of said DNA sequence in said transformed cells; and
recovering the polypeptide whose expression has been coded for by said sequence.

# FIGURE 1

# FIGURE 2

# FIGURE 3

# FIGURE 4

# FIGURE 5

## FIGURE 6

# FIGURE 7

FIGURE 8

## CC6 SEQUENCE

1                                                                                    50
CGAAATCACGCCTCCTCGCATTCTCACCAGGATGAACAAAAGGACACTCC
GCTTTAGTGCGGAGGAGCGTAAGAGTGGTCCTACTTGTTTTCCTGTGAGG


                                                                                    100
GTCCAATCATGCGTATACGCCCTCGAACAAGGCTTCACCTTAAAACTATA
CAGGTTAGTACGCATATGCGGGAGCTTGTTCCGAAGTGGAATTTTGATAT


                                                                                    150
CATCCTAAACTCATCACTCCCATACACTCCAACATTCATATCAGGCAACA
GTAGGATTTGAGTAGTGAGGGTATGTGAGGTTGTAAGTATAGTCCGTTGT


                                                                                    200
CTACCTCACTCACACACTCCTTTTCAACACACTGCGGAATCTCAGTCTCT
GATGGAGTGAGTGTGTGAGGAAAAGTTGTGTGACGCCTTAGAGTCAGAGA


ACACCCTCAAACCCTCCTAGCAAAACCCG
TGTGGGAGTTTGGGAGGATCGTTTTGGGC

## EUROPEAN SEARCH REPORT

European Patent Office

Application Number

| DOCUMENTS CONSIDERED TO BE RELEVANT | EP 91306899.5 |
|---|---|

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 82, no. 5, February 3, 1975 Columbus, Ohio, USA R.B. GUCHHAIT et al. "Acetyl coenzyme A carboxylase system of Escherichia coli. Purification and properties of the biotin carboxylase, carboxyltransferase, and carboxyl carrier protein components" page 21b, left column, abstract-no. 27 733w & J. Biol. Chem. 1974, 249 (20), 6633-45 -- | 1,4-7 | C 07 K 13/00<br>C 07 K  3/20<br>C 12 N 15/52<br>C 12 N 15/29 |
| D,Y | CHEMICAL ABSTRACTS, vol. 103, no. 15, October 14, 1985, Columbus, Ohio, USA B.J. NIKOLAU et al. "Use of streptavidin to detect biotin-containing proteins in plants" page 372, right column, abstract-no. 119 361d & Anal. Biochem. 1985, 149 (2), 448-53 -- | 5 | |
| D,Y | BIOCHEMICAL SOCIETY TRANSACTIONS, vol. 14, no. 1, February 1986, Oxford, UK A. HELLYER et al. "Plant acetyl-CoA carboxylase" pages 565-568 * Totality * ---- | 1,3-7 | TECHNICAL FIELDS SEARCHED (Int. Cl.5)<br><br>C 07 K<br>C 12 N |

The present search report has been drawn up for all claims

| Place of search | Date of completion of the search | Examiner |
|---|---|---|
| VIENNA | 15-10-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)